# EUROPEAN PATENT APPLICATION

(11) **EP 4 316 372 A1**
(43) Date of publication of application: **07.02.2024**
(21) Application number: 22188958.7
(22) Date of filing: 05.08.2022
(51) Int. Cl.: A61B 5/1477, A61B 5/1486

(54) **ELECTROCHEMICAL ANALYTE SENSOR HAVING IMPROVED REPRODUCIBILITY AND ASSOCIATED FABRICATION PROCESS**

(71) Applicant: PharmaSens AG, 4105 Biel-Benken (CH)
(72) Inventor: Bitterli, Joanna, 2074 Marin-Epagnier (CH); Skupienski, Radek, 2034 Peseux (CH); Hadvary, Paul, 4105 Biel-Benken (CH)
(74) Representative: Detken, Andreas

(57) **Abstract**

In a process for fabricating an electrochemical analyte sensor, a sensor substrate (101) that carries a working electrode (131) comprising a platinum black layer (104) is provided. An analyte sensing layer (105) is disposed onto the platinum black layer. In order to reproducibly obtain high sensitivity and long-term stability, the platinum black layer is activated before the analyte sensing layer is disposed onto the platinum black layer. For activation, the sensor substrate is exposed to ultraviolet radiation in an ozone-containing atmosphere.

## Description

### TECHNICAL FIELD

The present invention relates to a process for fabricating an electrochemical analyte sensor and to a sensor that is obtainable by such a fabrication process.

### PRIOR ART

Electrochemical analyte sensors are commonly used to measure the concentrations of analytes in vivo. In particular, implantable electrochemical glucose sensors for continuously monitoring the blood glucose level in an individual are known. The ultimate goal is to develop an "artificial pancreas", i.e., a system that combines continuous glucose monitoring with an insulin pump in a closed loop.

Reference [1] (van der Wal, P., Hadvary, P., Tschirky, H.-J and Rooij, N., "Flexprint Substrate Enzyme Sensor For Determination Of Daily Glucose-profiles Of Diabetic Patients", ECS Transactions. 50 (2013), pp. 89-92, DOI 10.1149/05012.0089ecst) discloses a miniaturized glucose sensor that can be easily implanted into a patient's body. The sensor comprises a flexprint-based substrate having a needle-like implantable tip portion, which is designed to be implanted with the aid of a guiding needle. At the distal end of the tip portion, three electrodes are arranged: a platinum working electrode, a counter electrode, and an Ag/AgCI reference electrode. The enzyme glucose oxidase is immobilized on the distal end of the tip portion. When contacted with glucose in the presence of molecular oxygen, the enzyme catalyzes oxidation of the glucose into gluconolactone and hydrogen peroxide. The hydrogen peroxide is then quantified by amperometric measurements. A diffusion-limiting membrane layer is employed to limit glucose diffusion towards the enzyme while not impeding oxygen diffusion, whereby the linear range of the sensor is increased even at low oxygen levels.

The sensitivity of an electrochemical analyte sensor is limited, inter alia, by the surface area of the working electrode. It is therefore desirable to provide a working electrode that has a high surface area.

US2020060586A1 discloses an amperometric glucose sensor having an electrode formed from platinum black, which has a high ratio of active surface area to geometric area. The platinum black is applied by an electrodeposition process that involves pulsed currents. An analyte sensing layer comprising the enzyme glucose oxidase is deposited over the platinum black layer. An analyte-modulating membrane layer is deposited over the analyte sensing layer to limit the amount of analyte that reaches the active enzyme.

GB933450 discloses a method of electrochemically depositing a solid platinum layer onto a metallic base layer, which may be made of gold.

Reference [2] (Kell, D.B. and Davey, C.L., "Conductimetric and impedimetric devices", In: Cass, A.E.G. (ed.), "Biosensors: A practical approach", Oxford University Press, Oxford (1990), pp. 125-152) discloses an electrodeposition process for depositing a platinum black layer onto a platinum base layer.

### SUMMARY OF THE INVENTION

Prior-art processes for manufacturing electrochemical analyte sensors suffer from poor reproducibility, the resulting sensors exhibiting large variations of sensitivity between sensors as well as unsatisfactory long-term stability. It is an object of the present invention to provide a fabrication process for an electrochemical analyte sensor that enables reproducible fabrication of a sensor with high sensitivity and good long-term stability.

This object is achieved by a process according to claim 1. Further embodiments of the invention are laid down in the dependent claims.

The present invention provides a process for fabricating an electrochemical analyte sensor, the process comprising the steps of:
providing a sensor substrate that carries a working electrode comprising a platinum black layer; and
depositing an analyte sensing layer onto the platinum black layer.

In order to improve reproducibility of sensitivity and long-term stability, the platinum black (Pt black) layer is activated before the analyte sensing layer is disposed onto the platinum black layer, activation comprising exposing the sensor substrate to ultraviolet radiation in an ozone-containing atmosphere.

In the invention, the platinum black layer is subjected to a UV-ozone surface treatment before the analyte sensing layer is applied. For UV-ozone surface treatment, an ozone-containing atmosphere is generated by irradiating a gas that contains molecular oxygen (usually air) with UV-C radiation, in particular, with ultraviolet radiation having a spectral component at a wavelength below 200 nm. An ultraviolet light source, typically in the form of a low-pressure mercury vapor discharge lamp, is used for that purpose, which generates UV-C radiation at wavelengths of approximately 185 nm and approximately 254 nm. The UV light not only generates ozone from molecular oxygen, but also breaks down organic contaminants on the surface. The procedure can produce near-atomically clean surfaces without causing damage to the sample. UV-ozone surface treatment is normally used for cleaning surfaces that are contaminated with organic contaminants. Surprisingly, the inventors have found that UV-ozone surface treatment massively improves reproducibility of the sensitivity and long-term stability of the sensor even if the platinum black layer is devoid of organic contaminants.

In contrast to other activation methods, e.g., oxygen plasma treatment, the UV-ozone surface treatment does not damage other parts of the sensor. In particular, the sensor substrate may further carry a reference electrode comprising a layer of silver and silver chloride (Ag/AgCI). Such a reference electrode would be rapidly damaged in a plasma treatment process. In contrast, subjecting the reference electrode to a UV-ozone treatment does not damage the reference electrode.

The sensor substrate may have a first side and a second side, the working electrode being disposed on the first side. The sensor substrate may further carry a counter electrode, which may also comprise a platinum black layer. The counter electrode may be disposed on the second side of the sensor substrate. It may then be desirable to also activate the platinum black layer of the counter electrode. To this end, during a first time period, the first side of the sensor substrate, including the working electrode, may be oriented towards the ultraviolet light source, and during a second time period, the second side of the substrate, including the counter electrode, may be oriented towards the ultraviolet light source. In other words, the process may comprise flipping the sensor substrate after some exposure time.

Preferably, the analyte sensing layer comprises a catalyst that catalyzes a reaction of the analyte. In particular, the catalyst may catalyze a reaction of the analyte with another compound, in particular, with oxygen. The catalyst may comprise an enzyme. The analyte may be glucose, and the catalyst may comprise an enzyme that catalyzes a reaction of glucose with oxygen. In particular, the catalyst may comprise glucose oxidase, more particularly, together with FAD as cofactor. The analyte sensing layer may further comprise at least one binding protein, in particular, an albumin. The enzyme may have been cross-linked with the binding protein by glutaraldehyde, and/or molecules of the binding protein may have been cross-linked with one another (see below).

In preferred embodiments, the analyte sensing layer is disposed onto the platinum black layer by dip coating. Experiments have shown that a more uniform analyte sensing layer can be obtained by dip coating than by other application processes such as spin coating. In a dip coating process, the sensor substrate is immersed in a precursor liquid, removed from the precursor liquid in a controlled manner, and dried.

The precursor liquid will generally comprise the catalyst that catalyzes the reaction of the analyte, in particular, an enzyme as discussed above. The precursor liquid may further comprise the binding protein and glutaraldehyde as an immobilizing agent. The viscosity of the precursor liquid can be controlled by the relative amounts of enzyme, protein, and glutaraldehyde. The binding protein may be an albumin and may be selected from the group of serum albumins. Preferably it is human serum albumin.

The precursor liquid may further comprise a surfactant to improve wetting of the platinum black surface by the precursor liquid during the dip-coating process. Preferably, the surfactant is selected from the group of the non-ionic octylphenol ethoxylates. The surfactant may be octoxynol-9, also known under the tradename Triton X-100^{™}.

The enzyme may be immobilized in the analyte sensing layer by causing cross-linking of the enzyme molecules with the binding protein, and/or by causing cross-linking of molecules of the binding protein with one another, using the glutaraldehyde as a cross-linking agent. Glutaraldehyde works as a cross-linking agent by linking the two reactive aldehyde groups of glutaraldehyde to one free amino group each of the enzyme and the binding protein or to two free amino groups of the binding protein.

Preferably, the analyte sensing layer is dried subsequently to dip-coating, using air with controlled temperature and humidity. Cross-linking takes place mainly during drying. The kinetics of the cross-linking process may be controlled by the drying conditions. Optimal adherence of the analyte sensing layer without the formation of cracks can be ensured by progressively increasing the temperature of the air, in particular, from a temperature below 15 °C to a temperature above 20 °C, while simultaneously reducing the relative humidity of the air, in particular, from a value above 70% to a value below 50%, over a time period of at least 5 hours.

The process may further comprise a step of depositing an analyte-modulating membrane layer onto the analyte sensing layer, the analyte-modulating membrane layer being configured to control (in particular, limit) diffusion of the analyte to the analyte sensing layer. The analyte-modulating membrane layer is preferably permeable to oxygen. In particular, the analyte-modulating layer may control diffusion of glucose while being permeable to oxygen. The analyte-modulating membrane layer may be applied by a dip-coating process as well.

The process may further comprise a step of depositing a biocompatible layer onto the analyte-modulating membrane layer, the biocompatible layer being configured to improve biocompatibility of the electrochemical analyte sensor. Again, the biocompatible layer may be applied by a dip-coating process.

The process may comprise a step of creating the platinum black layer of the working electrode and, optionally, of the counter electrode. In particular, the sensor substrate may comprise a structured gold layer, and the process may comprise:
depositing a solid platinum layer on a first portion of the structured gold layer by an electrochemical process; and
depositing the platinum black layer on the solid platinum layer to obtain the working electrode and, optionally, the counter electrode, by an electrochemical process.

The process may further comprise creating the Ag/AgCI layer of the reference electrode. This may be done by the following steps:
depositing an Ag layer on a second portion of the structured gold layer by an electrochemical process, said second portion being different and preferably electrically insulated from the first portion on which the platinum and platinum black layers are disposed; and
partially chlorinating the Ag layer by an electrochemical process to obtain an Ag/AgCl layer, the Ag/AgCl layer forming the reference electrode of the analyte sensor.

Experiments have shown that the order of creation of the Ag/AgCI layer and the platinum black layer is important for the morphology of the platinum black layer and for the sensitivity of the resulting sensor. Preferably, the platinum black layer is deposited onto the platinum layer only after the Ag/AgCl layer has been created. This order of deposition steps ensures high sensitivity of the resulting sensor. In contrast, if the Ag/AgCl layer is created only after the platinum black layer has been created, morphology of the platinum black layer may be negatively affected, with detrimental effects on sensor performance. The proposed sequence of deposition steps is advantageous even in cases where if the platinum black layer is not subjected to a UV-ozone surface treatment before the analyte sensing layer is applied.

During the electrodeposition steps, the first and second portions of the gold layer may be kept at different electric potentials to cause selective electrodeposition only to the desired portions of the sensor.

Specifically, the following two sequences of steps lead to good results:
Sequence A:
   1) electrodeposition of the Ag layer on the second portion of the gold layer;
   2) partial chlorination of the Ag layer to obtain the Ag/AgCl layer;
   3) electrodeposition of the Pt layer on the first portion of the gold layer; and
   4) electrodeposition of the Pt black layer on the Pt layer.
Sequence B:
   1) electrodeposition of the Pt layer on the first portion of the gold layer;
   2) electrodeposition of the Ag layer on the second portion of the gold layer;
   3) partial chlorination of the Ag layer to obtain the Ag/AgCl layer; and
   4) electrodeposition of the Pt black layer on the Pt layer.

These sequences are advantageous even if no UV-ozone surface treatment is carried out.

Depositing the Ag layer on the second portion of the gold layer may comprise: Immersing the sensor substrate in an aqueous plating solution comprising silver cyanide at a pH of at least 11 and electrodepositing the platinum onto the gold layer at a negative potential against a counter electrode. The negative potential may be applied in a continuous or pulsed manner. The plating solution may further comprise one or more of the following components: potassium cyanide, potassium carbonate and gloss-enhancing additives.

Partial chlorination of the Ag layer may be carried out using standard chlorination protocols in an aqueous KCI solution.

Depositing the platinum layer on the first portion of the gold layer may comprise: Immersing the sensor substrate in an aqueous solution of hydrogen hexahydroxoplatinate(IV) (H₂Pt(OH)₆) and potassium hydoxide (KOH) and electrodepositing the platinum onto the gold layer at a negative potential relative to an external silver/silver chloride (Ag/AgCI) reference electrode. For instance, a potential of -0.5 to -1.0 V, in particular, -0.7 V relative to the reference electrode may be applied, using a platinum counter electrode. The negative potential may be applied in a continuous or pulsed manner. The solid platinum layer may be a smooth, solid layer of platinum.

Depositing the platinum black layer on the solid platinum layer may comprise: Immersing the sensor substrate in an aqueous solution of platinum(IV)-chloride (PtCl₄), lead(II)-acetate trihydrate (Pb(CH₃CO₂)₂ x 3H₂O), and hydrochloric acid (HCI) and electrodepositing the platinum black onto the solid platinum layer at a negative potential relative to an external Ag/AgCI reference electrode. For instance, a potential of -0.1 to -0.3 V, in particular, -0.15 V relative to the reference electrode may be applied, using a platinum counter electrode. The negative potential may be applied in a continuous or pulsed manner.

The present invention also provides an electrochemical (in particular, amperometric) analyte sensor comprising a sensor substrate that carries a working electrode comprising a platinum black layer and an analyte sensing layer disposed on the platinum black layer, wherein the sensor is obtainable by the process of the present invention.

In addition to the working electrode, the electrochemical analyte sensor may comprise at least one of the following additional electrodes on the substrate:
a reference electrode, in particular, a reference electrode having a layer that comprises silver and silver chloride (i.e., an Ag/AgCl layer); and
a counter electrode for closing an electrical circuit with the working electrode and, where applicable, with the reference electrode, in particular, a counter electrode comprising a platinum layer and a platinum black layer disposed on the platinum layer.

The working electrode and reference electrode may be disposed on a first side of the substrate, and the counter electrode may be disposed on a second side of the substrate opposite to the first side. The reference electrode and the counter electrode may be covered with the analyte sensing layer as well, and the analyte-modulating membrane layer may cover the analyte sensing layer also in the region where the analyte sensing layer covers the reference and/or counter electrodes. The sensor may comprise more than one working electrode, more than one reference electrode, and/or more than one counter electrode.

The sensor substrate may have a proximal foot portion and a distal tip portion. The tip portion may be elongated and needle-shaped, defining a longitudinal direction. The tip portion may be configured to be implanted subcutaneously into the patient's body. It may have a length along the longitudinal direction of 5 to 30 mm, preferably 10 to 20 mm, and lateral dimensions perpendicular to the longitudinal direction below 500 µm, preferably below 300 µm, more preferably below 200 µm. The ratio of length to width of the tip portion is preferably at least 50. The working electrode, reference electrode and counter electrode may be disposed on the sensor substrate near the distal end of the tip portion, in a distal end portion thereof. The distal end portion may have a length that is between 1% and 50%, in particular, between 1% and 20% of the length of the tip portion. In absolute numbers, the distal end portion may have a length between 0.1 and 15 mm, in particular, between 0.1 and 5 mm. The foot portion will generally be wider than the tip portion, in particular, by a factor of at least 10. In absolute numbers, the foot portion may have a width between 1 and 20 mm, preferably between 2 and 10 mm. An intermediate shaft portion may connect the foot portion to the tip portion. The shaft portion may have a width that is smaller than the width of the foot portion, but larger than the width of the tip portion in order to improve mechanical stability of the sensor.

The electrochemical analyte sensor may comprise contact pads that are disposed on the sensor substrate, the contact pads being provided for establishing electrical contact to electronic circuitry for operating the electrochemical analyte sensor. The contact pads may be disposed on the foot portion of the sensor substrate. They may be disposed on only one single side of the sensor substrate or on both sides thereof. At least one contact pad or all contact pads may comprise a via through the sensor substrate, i.e., a through-hole through the sensor substrate that is plated with conductive material to establish an electrical connection between the two sides of the sensor surface.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows an exemplary implantable glucose sensor in a top view;
- Fig. 2: shows an enlarged view of the tip portion of the glucose sensor in Fig. 1 in a top view;
- Fig. 3: shows the glucose sensor in Fig. 1 in a bottom view;
- Fig. 4: shows an enlarged view of the tip portion of the glucose sensor in Fig. 1 in a bottom view;
- Fig. 5: shows a scanning electron microscope (SEM) image of a platinum black layer in a top view, the platinum black layer having been applied after fabrication of the Ag/AgCI reference electrode;
- Fig. 6: shows an SEM image of a platinum black layer in a top view, the platinum black layer having been applied before fabrication of the Ag/AgCI reference electrode;
- Figs. 7A-7E: illustrate a process for fabricating the working electrode and the counter electrode of a glucose sensor according to an embodiment of the invention;
- Figs. 8A-8C: illustrate a process for fabricating the reference electrode of a glucose sensor according to an embodiment of the invention;
- Fig. 9: is a flow diagram illustrating a preferred sequence of steps in the process for fabricating a glucose sensor according to Figs. 7A to 7E and Figs. 8A to 8C;
- Fig. 10: illustrates a UV-ozone cleaning apparatus;
- Figs. 11A-11D: show diagrams illustrating measurements of sensor response as a function of H₂O₂ concentration for sensors without an analyte sensing layer whose platinum black layer has not been activated (Figs. 11A and 11C) and of sensors whose platinum black layer has been activated by UV-ozone treatment (Figs. 11B and 11D), on day 1 after first use (Figs. 11A and 11B), and on day 4 after first use (Figs. 11C and 11D);
- Fig. 12: shows a diagram illustrating measurements of sensor response as a function of glucose concentration for sensors with an analyte sensing layer whose platinum black layer has not been activated (lower trace, open triangles) and of sensors whose platinum black layer has been activated by UV-ozone treatment (closed circles), on day 1 after first use; and
- Fig. 13: shows a diagram illustrating measurements of sensor response as a function of H₂O₂ concentration for sensors without an analyte sensing layer with different orders of fabrication steps.

### DESCRIPTION OF PREFERRED EMBODIMENTS

### Setup of an exemplary implantable electrochemical analyte sensor

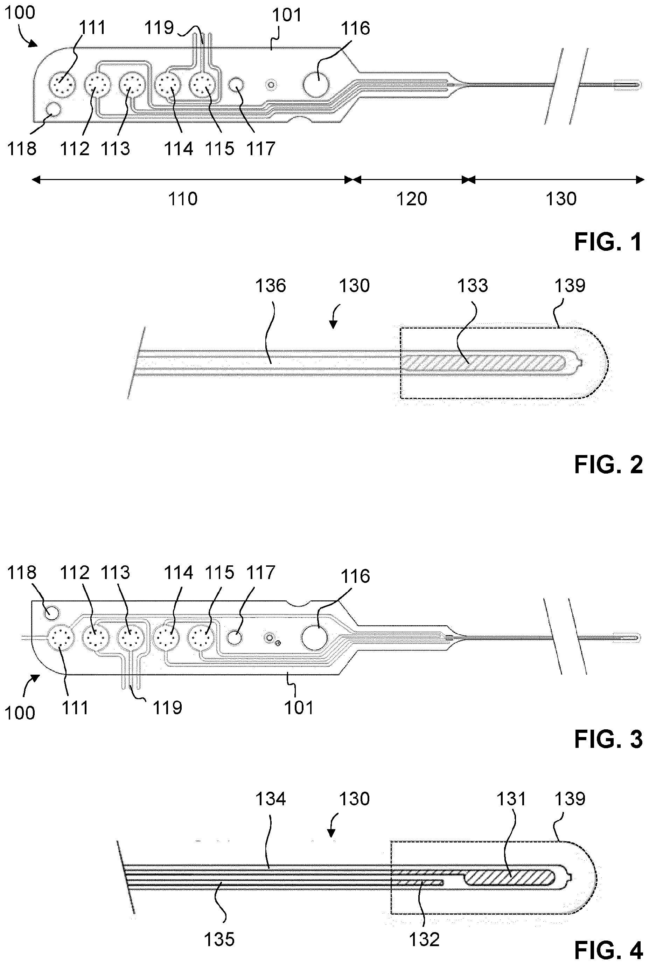

Figures 1 to 4 illustrate an exemplary embodiment of an electrochemical analyte sensor 100 for implantation into a patient's body. In the present example, the electrochemical analyte sensor 100 is a glucose sensor.

The electrochemical analyte sensor 100 comprises a sensor substrate 101, which carries a plurality of electrodes, conductive traces and contact pads, as will be explained in more detail below. The sensor substrate is made of a flexible base material, for instance a liquid crystal polymer (LCP) or a polyimide like Kapton^{™}. The base material can be as thin as 50 µm. The sensor substrate with electrodes, conductive traces and contact pads may be produced using technologies known per se for fabricating flexible printed circuit boards (flexible PCBs or "flexprints").

The sensor substrate 101 has three distinct portions: a foot portion 110, a shaft portion 120, and a sensor tip portion 130. The foot portion 110 defines a proximal end of the sensor substrate 101. It has a width of approximately 3 mm and a length of approximately 12 mm. The shaft portion 120 connects the tip portion 130 to the foot portion 110. This portion is somewhat narrower than the foot portion, having a width of approximately 1 mm and a length of approximately 4 mm. The tip portion has a width of only approximately 150 µm and a length of approximately 15 mm. In a distal end portion 139 of the tip portion 130, the sensor substrate 101 carries three sensing electrodes: a working electrode 131, a reference electrode 132, and a counter electrode 133. The distal end portion 139 is adapted to be implanted subcutaneously into the patient's body such that the sensing electrodes are in contact with subcutaneous interstitial fluids. The remainder of the tip portion 130 that is proximal of the end portion 139 is protected by a non-conducting polymer, e.g., by a solder stop layer. The distal end portion 139 remains free of the solder stop layer.

The counter electrode 133 is disposed on the top surface of the tip portion 130 (see Figs. 1 and 2), whereas the working electrode 131 and the reference electrode 132 are disposed on the bottom surface of the tip portion 130 (see Figs. 3 and 4).

In the foot portion 110, several contact pads 111, 112, 113, 114 and 115 are provided. All contact pads are formed as vias through the sensor substrate and can therefore be contacted from both sides of the sensor substrate 101. A conductive trace 134 on the bottom of substrate 101 connects working electrode 131 to contact pad 111. A further conductive trace 135 on the bottom of substrate 101 connects reference electrode 132 to contact pad 115. A conductive trace 136 on the top of substrate 101 connects counter electrode 133 to contact pad 113. Contact pads 112 and 114 are connected to conductive traces on the foot portion 110 and shaft portion 120 for shielding conductive traces 135 and 136 from external electromagnetic fields. Alignment holes 116, 117 and 118 serve for aligning the sensor substrate 101 with an external mounting structure.

Additional conductive traces 119 are visible in Figs. 1 and 3. These additional conductive traces are only used during production of the electrochemical analyte sensor 100. During production, a plurality of identical electrochemical analyte sensors are formed by patterning electrodes, contact pads and conductive traces onto a common sheet of substrate material. Similar contact pads of all sensors on the sheet are connected to a common contact node by one of the conductive traces 119 each. In particular, the contact pads 115 of all sensors on the sheet, which serve for contacting the reference electrode 132, are connected to a common contact node by one of the conductive traces 119. In this manner, during production a common potential can readily be applied to the reference electrodes 132 of all sensors on the sheet. This may be used for applying an Ag/AgCI coating to the reference electrode by an electrochemical deposition method. After production of the sensors on the sheet is finished, the sensors are individualized by punching, and the conductive traces 119 may be cut away, as they are not used any more.

The working electrode 131, the reference electrode 132, and the counter electrode 133 form a three-electrode electrochemical sensor, as it is well known per se. The working electrode 131 is the electrode where the reaction of interest occurs. The reference electrode 132 is an electrode that has a stable and well-known electrode potential. The counter electrode 133 is used to close the current circuit.

The sensor may be operated using electronic circuitry configured as a potentiostat for applying a well-defined potential difference between the working electrode 131 and the reference electrode 132 while minimizing a current through the reference electrode 132, and for measuring a current that flows through the working electrode 131 and/or through the counter electrode 133. Suitable potentiostats are well known in the art.

### Electrode fabrication

The three electrodes 131, 132, 133 are fabricated by electrochemical deposition of the electrode material onto a gold layer, which was applied to the substrate 101 and structured by processes known per se in the field of flexprint fabrication.

Preferably, the reference electrode is fabricated first. Specifically, the reference electrode 132 is fabricated by electrochemical deposition of a silver layer onto a corresponding portion of the structured gold layer, followed by partial chlorination, to obtain a silver/silver chloride (Ag/AgCI) layer, preferably having a thickness of 5-10 µm, e.g., 8 µm. The electrochemical deposition process is well known, and electrolytic baths for this process are available commercially. Specifically, an aqueous plating solution comprising silver cyanide at a pH of at least 11 may be used for electrodepositing the platinum onto the gold layer, using well-known deposition protocols. An aqueous KCI solution may be used for subsequent chlorination, using well-known chlorination protocols. The portions of the structured gold layer that are to remain free of Ag/AgCl are protected from Ag deposition by applying appropriate electric potentials to these portions, as it is well known in the art.

The working electrode 131 and the counter electrode 133 are subsequently fabricated by electrochemical deposition of a smooth, solid layer of platinum, preferably having a thickness of 100 to 300 nm, onto the corresponding portions of the structured gold layer, followed by electrochemical deposition of a layer of platinum black onto the solid platinum layer, preferably having a thickness of 1 to 3 µm. The solid platinum layer may be deposited onto the gold layer by the following protocol: Immersing the sensor substrate in a solution of hydrogen hexahydroxoplatinate(IV) (H₂Pt(OH)₆) and potassium hydoxide (KOH) and electrodepositing the platinum onto the gold surfaces for a defined period of time at a DC potential of -0.7 V against an external silver/silver chloride (Ag/AgCI) reference electrode using a platinum counter electrode. Depositing the platinum black layer onto the platinum layer may comprise: Immersing the sensor substrate in a solution of platinum(IV)-chloride (PtCl₄), lead(II)-acetate trihydrate (Pb(CH₃CO₂)₂ x 3H₂O), and hydrochloric acid (HCI) and electrodepositing the platinum black onto the platinum surfaces for a defined period of time at a DC potential of -0.15 V against an external silver/silver chloride (Ag/AgCI) reference electrode using a platinum counter electrode. Other protocols as they are known in the art may be used as well.

The resulting layer of platinum black has extremely high surface area ratio (SAR), the surface area ratio being defined as the ratio between active surface area and geometric area of the electrode.

### Dependence of morphology of platinum black on order of fabrication steps

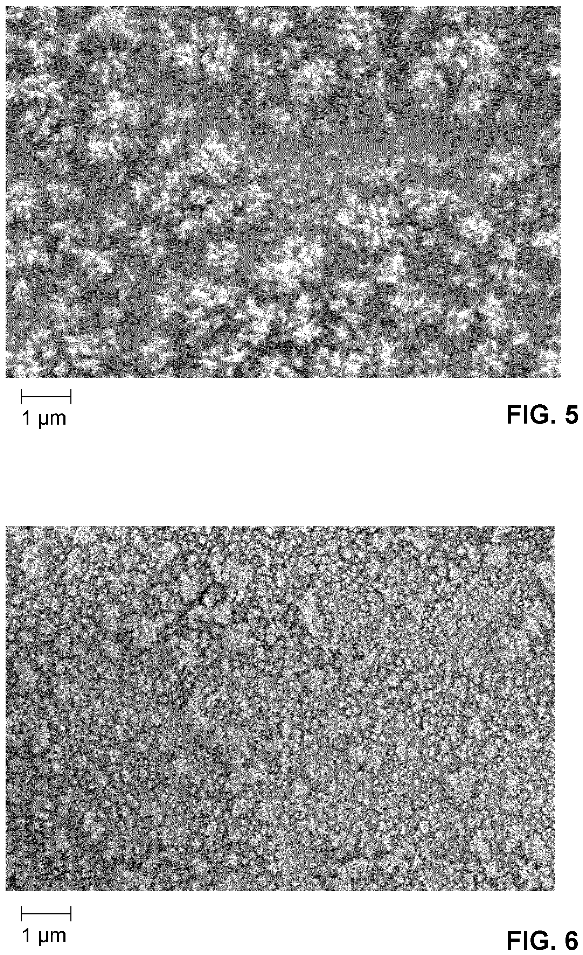

Figures 5 and 6 illustrate that the morphology of the platinum black layer strongly depends on whether the platinum black layer is created before or after the Ag/AgCl layer.

The morphology shown in Fig. 5 was obtained using the above-described sequence, wherein the Ag/AgCl layer was created first and the Pt and Pt black layers were deposited only thereafter. The Pt black layer exhibits a complex dendritic morphology, leading to very large active surface area.

In contrast, the morphology shown in Fig. 6 was obtained using a sequence wherein the Ag/AgCl layer was created only after deposition of the Pt black layer. Surprisingly, the morphology of the Pt black layer is completely different than the morphology in Fig. 5, exhibiting much reduced complexity and reduced active surface area. This indicates that the conditions under which the Ag/AgCl reference electrode is created are detrimental to the quality of the Pt black layer.

### Activation of platinum black by UV-ozone treatment

The platinum black layer is subsequently coated with an analyte sensing layer comprising the enzyme glucose oxidase, acting as a catalyst for the reaction of glucose with oxygen to form hydrogen peroxide, to enable electrochemical (to be precise, amperometric) detection of glucose.

Before being coated with the analyte sensing layer, the platinum black layer is activated by exposing the platinum black layer to UV radiation in the presence of ozone, using a commercially available UV-ozone cleaning apparatus. The UV-ozone cleaning apparatus may comprise a low-pressure mercury vapor gas discharge lamp, which generates UV-C radiation at wavelengths of approximately 185 nm and approximately 254 nm. The 185-nm UV light dissociates molecular oxygen O₂ into triplet atomic oxygen O(³P). Triplet atomic oxygen O(³P) combines with molecular oxygen O₂ and generates ozone O₃. The 254-nm UV light dissociates ozone O₃ and forms molecular oxygen O₂ and singlet atomic oxygen O(¹D). Singlet atomic oxygen O(¹D) has strong oxidation power.

Activation using the UV-ozone cleaning apparatus may be carried out using the following protocol: During a first time period, the top side of substrate, including the platinum black layer on the counter electrode, is oriented towards the lamp; during a second time period, the bottom side of the substrate, including the platinum black layer on the working electrode, is oriented towards the lamp. Each time period may last, e.g., between 1 and 30 min. In practice, good results are achieved with a duration of 5 min for each time period.

### Application of an analyte sensing layer

Subsequently, the analyte sensing layer is applied to the distal end portion 134, including the activated platinum black layers on the working electrode and on the counter electrode. This is preferably done by dip-coating. To this end, a viscous aqueous precursor liquid comprising the enzyme is provided. The substrate with the platinum black layer is immersed in the precursor liquid, slowly retracted and allowed to dry in dry and cool air. Thereby, a well-defined analyte sensing layer is formed on the distal end portion 134, the enzyme being entrapped in the analyte sensing layer.

### Dip-coating protocol

In an exemplary embodiment, the precursor liquid comprises a binding protein, in particular, human serum albumin, and glutaraldehyde as a cross-linking agent for immobilizing the enzyme in the analyte sensing layer by cross-linking. Specifically, by virtue of its two reactive aldehyde groups, the cross-linking agent links one free amino group each of the enzyme and the protein to each other, thus forming covalent bonds between the enzyme and the protein. In addition, the cross-linking agent also links free amino groups of the same or different molecules of the binding protein to one another, as it is well known in the art.

The term "binding protein" is used herein to distinguish said protein from the enzyme (which is a protein as well), and to emphasize that said protein is used to immobilize or "bind" the enzyme in the analyte sensing layer.

The viscosity of the precursor liquid can be controlled by the relative amounts of enzyme, binding protein, and glutaraldehyde.

The precursor liquid may further comprise a non-ionic surfactant, such as Triton X-100, to improve wetting of the platinum black surface.

During drying, the enzyme is conjugated to the protein via the glutaraldehyde, whereby the enzyme is immobilized in the layer.

The analyte sensing layer is preferably dried in air having controlled temperature and humidity, the air being agitated by a fan. In a preferred embodiment, the total drying time is 8-10 hrs. During this time, temperature of the air is increased from 10 °C to 23 °C, while its relative humidity is progressively reduced from 75% to 30%. In this manner, excellent adherence of the analyte sensing layer to the Pt black layer can be ensured, while the formation of cracks can be avoided. However, the drying conditions may vary. Good results are achieved if the temperature of the air is increased from a temperature below 15 °C to a temperature above 20 °C, and the relative humidity is concomitantly reduced from a value above 70% to a value below 50%, over a period of at least 5 hours.

A preferred protocol is as follows:

| Dipping time / min | 15' |
|---|---|
| Movement time for retraction from dipping bath / min:ss | 2':30" |
| Dipping temperature / °C | 10 |
| Drying time / min | Overnight |
| Drying start temperature / °C | 10 |
| Drying start humidity / %rel. humidity | 75 |
| Drying end temperature / °C | 23 |
| Drying end humidity / %rel. humidity | 30 |
| Number of dipping cycles | 1 |

Instead of human serum albumin, any serum albumin or any kind of soluble protein may be used as thickening agent.

Instead of Triton X-100, any other kind of non-ionic surfactants (like alcohols, ethers, ethoxylates), anionic surfactants (like carboxylates, sulfonates, sulphates), or cationic surfactants (like quaternary ammonium groups) may be used.

### Alternative composition of precursor liquid

In an alternative embodiment, the precursor liquid comprises a monomer, an initiator to start polymerization of the monomer, a polymerization catalyst, and a cross-linking agent. Specifically, the monomer may be acrylamide, the initiator may be ammonium persulfate (APS), which acts as a free radical initiator, the polymerization catalyst may be tetramethylethylenediamine (TEMED), and the cross-linking agent may be N,N-methylenebisacrylamide.

### Application of additional layers

An analyte-modulating membrane layer may be disposed onto the analyte sensing layer. The membrane layer modulates diffusion of the analyte to the analyte sensing layer. In particular, the membrane layer controls diffusion of glucose to the analyte sensing layer, while being permeable for oxygen.

In particular, the analyte-modulating membrane layer may comprise carboxylated polyvinyl chloride (PVC), 3-aminopropyl-trimethoxysilane, hydrophilic aliphatic polyether-based thermoplastic polyurethanes, or aliphatic and aromatic polycarbonate-based thermoplastic poly-urethanes. An example for a suitable carboxylated PVC membrane is disclosed in Benmakroha et al., Analyst 121 (1996), 521-526; and example for a suitable polysilane membrane is disclosed in Ward et al., Biosensors & Electronics 17 (2002) 181-189.

Depending on the material and the associated properties of the analyte-modulating membrane layer, a biocompatible layer may be disposed onto the analyte-modulating membrane layer as a final coating. The biocompatible layer improves biocompatibility of the electrochemical analyte sensor.

In particular, the biocompatible layer may comprise hydrophilic aliphatic polyether-based thermoplastic polyurethanes, or aliphatic and aromatic polycarbonate-based thermoplastic poly-urethanes.

The analyte-modulating membrane layer and the biocompatible layer may also be applied by dip-coating.

### Illustration of complete fabrication process

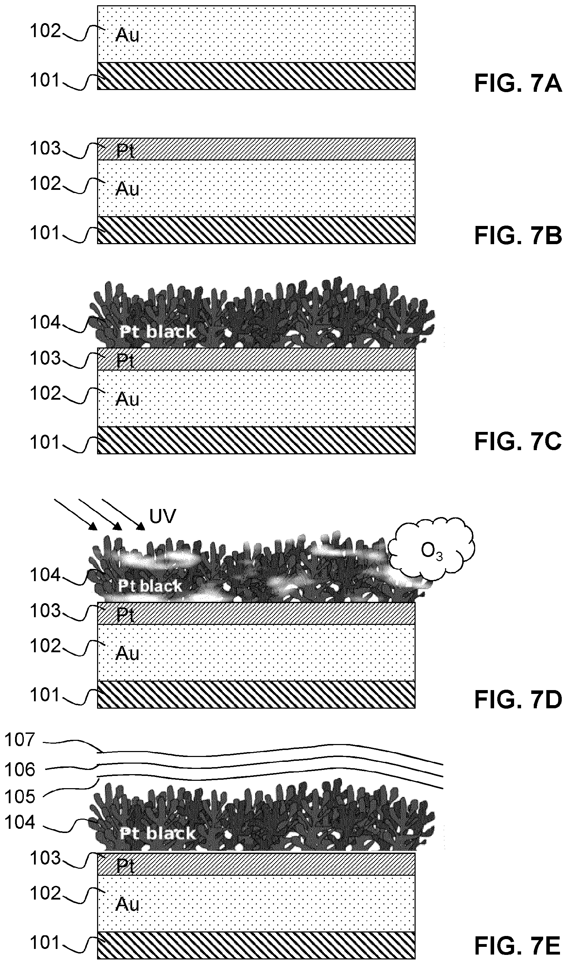

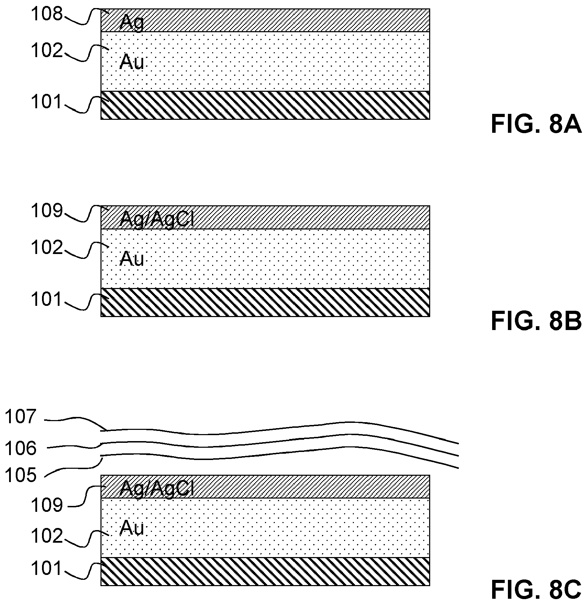

The above-described fabrication process is schematically illustrated in Figs. 7A-7E (working electrode and counter electrode) and Figs. 8A-8C (reference electrode). It is to be understood that these drawings are not to scale.

Figs. 7A to 7E illustrate fabrication of the working electrode and counter electrode. In Fig. 7A, a substrate 101 carrying a structured gold layer 102 is provided. In Fig. 7B, a platinum layer 103 is electrodeposited onto the first portion of the structured gold layer 102 in the manner described above. In Fig. 7C, a platinum black layer 104 is electrodeposited onto the platinum layer 103 in the manner described above. In Fig. 7D, the platinum black layer 104 is activated by irradiating the platinum black layer with UV radiation in the presence of ozone, as described above. In Fig. 7E, the activated platinum black layer 104 is coated with an analyte sensing layer 105, an analyte-modulating membrane layer 106, a biocompatible layer 107, as described above.

Figs. 8A to 8C illustrate fabrication of the reference electrode. In Fig. 8A, an Ag layer 108 is electrodeposited onto the second portion of the structured gold layer 102. In Fig. 8B, the Ag layer 108 is partially chlorinated to obtain an Ag/AgCl layer 109. In Fig. 8C, the Ag/AgCl layer 109 is covered by the analyte sensing layer 105, the analyte-modulating membrane layer 106, and the biocompatible layer 107 as well.

As discussed above, it is preferred that the steps that are illustrated in Figs. 8A and 8B are carried out before the step that is illustrated in Fig. 7C.

### Flow diagram

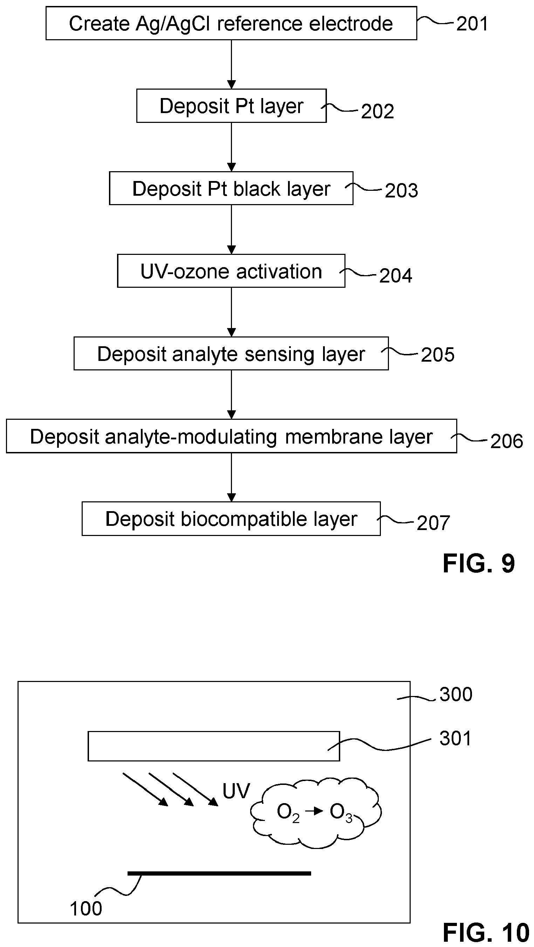

Figure 9 shows a flow diagram of the preferred sequence of steps of the fabrication process. In step 201, the Ag/AgCl layer is created on the second portion of the structured gold layer to form the reference electrode. In step 202, the Pt layer is deposited on the first portion of the structured gold layer, which is to form the working electrode and the counter electrode. In step 203, the Pt black layer is deposited on the Pt layer. In step 204, the Pt black layer is activated by UV-ozone treatment. In step 205, the analyte sensing layer is deposited on the sensor tip. In step 206, the analyte-modulating membrane layer is deposited, and in step 207, the biocompatible layer is deposited.

### Illustration of UV-ozone cleaning apparatus

Figure 10 illustrates, in a highly schematic manner, a UV-ozone cleaning apparatus 300. The UV-ozone cleaning apparatus comprises a UV source in the form or a large-area, low-pressure mercury gas discharge lamp 301 for generating UV radiation. The apparatus 300 is filled with an atmosphere that contains molecular oxygen, in particular, with air. The UV radiation converts molecular oxygen O₂ into ozone O₃. One or more sensors 100 are exposed to the UV radiation in the presence of the ozone-containing atmosphere. After a certain exposure time, the sensors may be flipped so as to expose both sides of each sensor to direct UV radiation.

### Improved reproducibility and stability

### a) Sensitivity to hydrogen peroxide

Reproducibility and sensor stability were tested for sensors whose platinum black layer had been activated by UV-ozone exposure as well as for sensors for which such an activation step had been omitted. To this end, eight sensors were manufactured as described above, without applying the analyte sensing layer, the analyte-modulating membrane layer and the biocompatible layer. For four of these sensors (the "non-activated sensors"), the activation step was omitted. For the other four sensors (the "UV-O3 activated sensors"), the platinum black layers were activated by UV-ozone exposure as described above. For each sensor, the current response to H₂O₂ concentration in physiological phosphate-buffered saline (PBS) solution was measured on days 1 and 4 after the sensors were first exposed to the PBS solution. The sensors were kept in the PBS solution between the measurements. An electrode potential of the working electrode of 0.6 V relative to the reference electrode was used for all measurements. The current through the working electrode was measured for six different H₂O₂ concentrations between 0 and 2 mM.

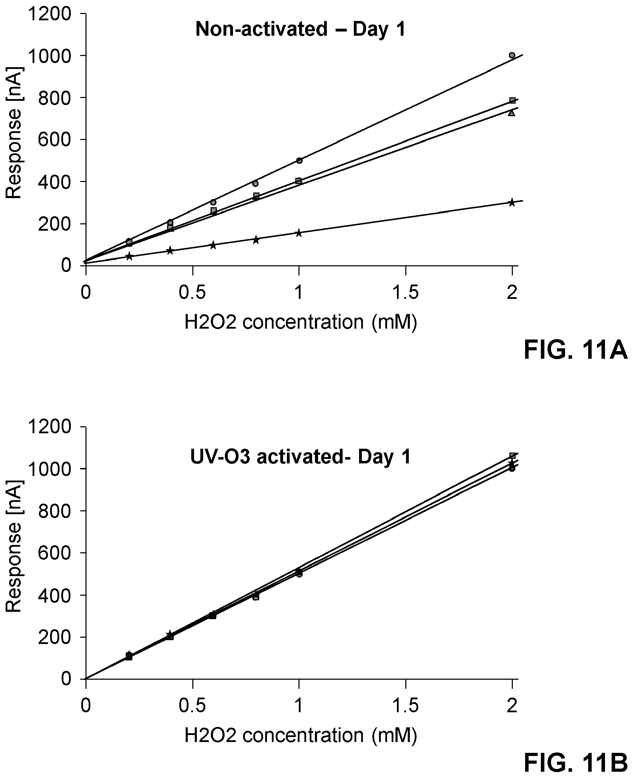

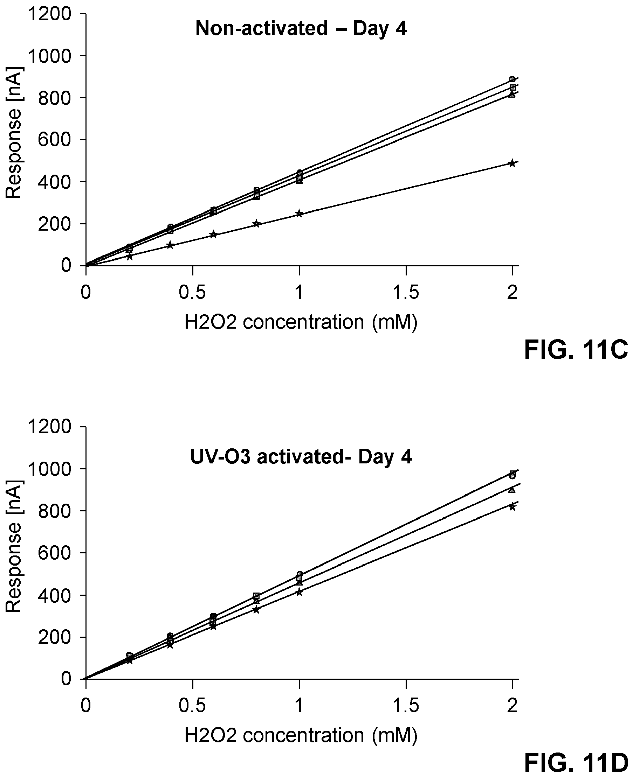

The results are shown in Figs. 11A-11D. The response to H₂O₂ concentration was approximately linear for all sensors in the measured concentration range. However, as apparent from Fig. 11A, the sensitivity on day 1 varied between the non-activated sensors by a factor of more than 3. In contrast, as apparent from Fig. 11B, the activated sensors exhibited much lower sensor-to-sensor variations, in the range of only a few percent. On day 4, the sensitivities of the non-activated sensors still varied almost by a factor of 2 (see Fig. 11C), while the activated sensors still exhibited much lower variations of sensitivities (see Fig. 11D). This shows that the activation step massively improved reproducibility of the sensor performance between sensors.

The data also show that stability is improved by the activation step. The non-activated sensors exhibited much larger sensitivity changes between days 1 and 4 than the activated sensors. In particular, three of the non-activated sensors exhibited a reduction of sensitivity by a few percent, as might be expected, while one of these sensors showed a marked increase of sensitivity by almost 50% (see Figs. 11A and 11C). Such spurious behavior makes it impossible to reliably predict changes of sensitivity over time in a physiological environment. Such a prediction is required because in practice the sensor cannot be recalibrated any more once it has been implanted, recalibration being difficult and inconvenient for the patient. In contrast, all of the activated sensors exhibited a moderate reduction of sensitivity over time, which can be easily predicted and taken into account during signal processing (see Figs. 11B and 11D).

### b) Sensitivity to glucose

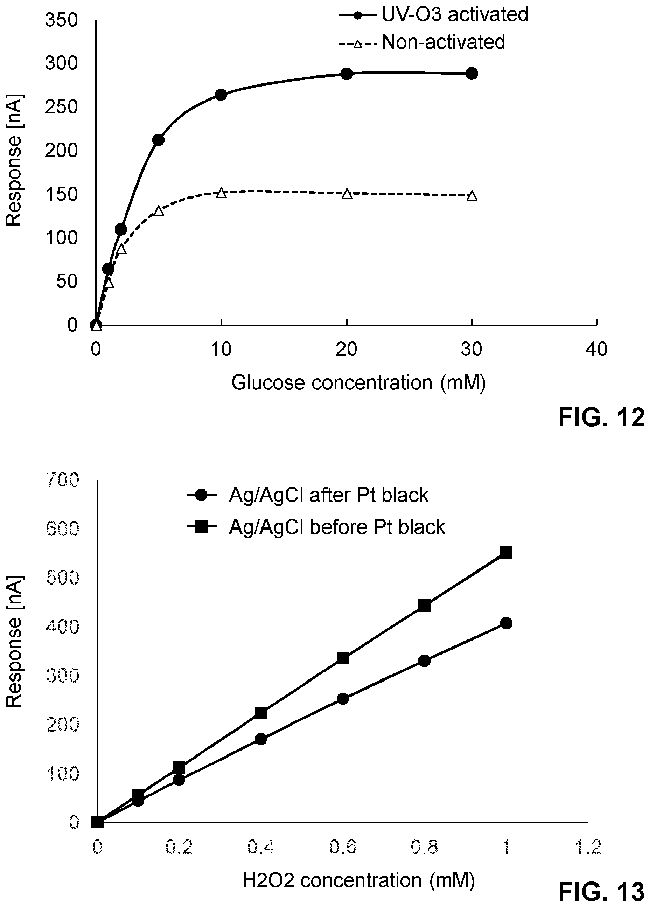

Figure 12 shows the results of measurements of sensitivity to glucose for two different sensors. The sensors were produced according to the protocol described above, including application of the analyte sensing layer, but without the analyte-modulating membrane layer and the biocompatible layer. For one of the sensors, the UV-ozone activation step was omitted, while for the other sensor, the UV-ozone activation step was included in the protocol.

The results show that sensitivity to glucose is markedly increased for the sensor whose Pt black layer has been activated.

### Alternatives to UV-ozone activation

Alternative activation protocols for the platinum black layers were tested. In particular, the platinum black layer was washed with isopropanol instead of carrying out a UV-ozone treatment. Washing with isopropanol led to inferior results. As another potential alternative, the platinum black layer was activated by oxygen plasma treatment. While this led to good reproducibility, the oxygen plasma caused damage to the Ag/AgCl reference electrode.

UV-ozone activation thus is the method of choice for obtaining a highly efficient working electrode with excellent reproducibility without damaging the reference electrode.

### Importance of order of fabrication steps

Figure 13 illustrates the results of measurements of sensitivity to H₂O₂ concentration for two sensors without an analyte sensing layer, analyte-modulating membrane layer and biocompatible layer. The first sensor was produced by first depositing the Pt and Pt black layers of the working electrode and counter electrode, followed by creation of the Ag/AgCl layer of the reference electrode. The corresponding sensor response is illustrated by black circles. The second sensor was produced by first creating the Ag/AgCl layer of the reference electrode and only thereafter depositing the Pt and Pt black layers of the working electrode and counter electrode. Apart from the order of the deposition steps, the fabrication conditions were identical for both sensors. The corresponding sensor response is shown by black squares. It can be clearly seen that the second sensor exhibited much better sensitivity than the first sensor. This highlights that the sequence of fabrication steps is important not only for the morphology of the Pt black layer, as discussed above in connection with Figs. 5 and 6, but also for the sensor performance.

## Claims

1. A process for fabricating an electrochemical analyte sensor, the process comprising the steps of:
providing a sensor substrate (101) that carries a working electrode (131) comprising a platinum black layer (104); and
depositing an analyte sensing layer (105) onto the platinum black layer (104),
**characterized in that** the process comprises a step of activating the platinum black layer (104) before the analyte sensing layer (105) is disposed onto the platinum black layer (104), activation comprising exposing the sensor substrate (101) to ultraviolet radiation in an ozone-containing atmosphere.

2. The process of claim 1, wherein the ozone-containing atmosphere is generated by irradiating an oxygen-containing gas with said ultraviolet radiation, the ultraviolet radiation having at least one spectral component at a wavelength below 200 nm.

3. The process of claim 1 or 2,
wherein the sensor substrate (101) has a first side and a second side,
wherein the working electrode (131) is disposed on the first side of the sensor substrate (101);
wherein the sensor substrate (101) further carries a counter electrode (133) comprising a second platinum black layer (104), the counter electrode (133) being disposed on the second side of the sensor substrate (101);
wherein the ultraviolet radiation is generated by an ultraviolet light source (301), in particular, by a gas discharge lamp;
wherein during a first time period, the first side of the sensor substrate (101) with the working electrode (131) is oriented towards the ultraviolet light source (301), and
wherein during a second time period, the second side of the substrate (101) with the counter electrode (133) is oriented towards the ultraviolet light source (301).

4. The process of any one of the preceding claims, wherein the sensor substrate (101) further carries a reference electrode (132) comprising an Ag/AgCl layer, the reference electrode (132) being exposed to the ozone-containing atmosphere during said step of activating the platinum black layer (104).

5. The process of any one of the preceding claims, wherein the analyte sensing layer (105) is applied to the platinum black layer (104) by dip coating.

6. The process of any one of the preceding claims, wherein the analyte sensing layer (105) comprises a catalyst that catalyzes a reaction of an analyte with molecular oxygen.

7. The process of claim 6,
wherein the catalyst comprises an enzyme,
in particular, wherein the analyte is glucose, and wherein the catalyst comprises an enzyme that catalyzes a reaction of glucose with molecular oxygen,
more particularly, wherein the catalyst comprises glucose oxidase.

8. The process of claims 5 and 7, wherein dip coating comprises immersing the sensor substrate (101) in a precursor liquid comprising the enzyme and a binding protein, in particular, an albumin, and glutaraldehyde, the enzyme and the binding protein being capable of being cross-linked by glutaraldehyde.

9. The process of claim 8, wherein the precursor liquid further comprises a surfactant, in particular, a surfactant selected from the group of the non-ionic octylphenol ethoxylates.

10. The process of claim 8 or 9, wherein the analyte sensing layer is dried subsequently to dip coating to cause cross linking, using air with controlled temperature and humidity, wherein temperature of the air is progressively increased while relative humidity of the air is progressively reduced.

11. The process of any one of the preceding claims, further comprising a step of:
depositing an analyte-modulating membrane layer (106) onto the analyte sensing layer (105), the analyte-modulating membrane layer (106) being configured to limit diffusion of the analyte to the analyte sensing layer (105).

12. The process of claim 11, further comprising a step of:
depositing a biocompatible layer (107) onto the analyte-modulating membrane layer (106), the biocompatible layer (107) being configured to improve biocompatibility of the electrochemical analyte sensor.

13. The process of any one of the preceding claims, wherein the sensor substrate (101) comprises a structured gold layer (102), and wherein the process comprises:
depositing a solid platinum layer (103) on a first portion of the structured gold layer (102); and
depositing the platinum black layer (104) on the solid platinum layer (103) to obtain the working electrode (131).

14. The process of claim 13, further comprising:
depositing an Ag layer (108) on a second portion of the structured gold layer (102); and
partially chlorinating the Ag layer (108) by an electrochemical process to obtain an Ag/AgCl layer (109), the Ag/AgCl layer (109) forming a reference electrode (102) of the analyte sensor,
wherein the Ag/AgCl layer (109) is created before the platinum black layer (104) is disposed on the solid platinum layer (103).

15. An electrochemical analyte sensor comprising a sensor substrate (101) that carries a working electrode (131) comprising a platinum black layer (104), the electrochemical analyte sensor further comprising an analyte sensing layer (105) disposed on the platinum black layer (104),
**characterized in that** the electrochemical analyte sensor is obtainable by the process of any one of the preceding claims.
